# EUROPEAN PATENT SPECIFICATION

(11) **EP 2 137 212 B1**
(45) Date of publication and mention of the grant of the patent: **03.06.2015**
(21) Application number: 08736345.3
(22) Date of filing: 17.04.2008
(51) Int. Cl.: C07K 14/395, C12H 1/14, C12P 21/06

(54) **Preparation of mannoprotein solutions**
Herstellung von Mannoproteinlösungen
Préparation de solutions de mannoprotéines

(30) Priority: 20.04.2007 EP 07106635; 04.10.2007 EP 07117898; 12.11.2007 EP 07120458
(43) Date of publication of application: 30.12.2009
(73) Proprietor: Rymco International AG, 6301 Zug (CH)
(72) Inventor: LANKHORST, Peter, Philip, NL-3051 JE Rotterdam (NL); BIJL, Hendrik, Louis, NL-3131 ZD Vlaardingen (NL); ÖZKAN, Ibrahim, NL-3087 EK Rotterdam (NL)
(74) Representative: Orr, Robert
(86) International application number: PCT/EP2008/054691
(87) International publication number: WO 2008/128973

(56) References cited:
- EP-A- 1 094 117
- WO-A-01/46380
- WO-A-96/13571
- WO-A-97/05272
- WO-A-2006/067145
- WO-A-2006/067147
- DUPIN ET AL: "Saccharomyces cerevisiae mannoproteins that protect wine from protein haze: Evaluation of extraction methods and immunolocalization", JOURNAL OF AGRICULTURAL AND FOOD CHEMISTRY, vol. 48, 2000, pages 1086-1095, XP001091139,

## Description

### Field of the invention

The present invention relates a method to produce a mannoprotein solution and to its use in wine stabilisation.

### Background of the invention

The presence of tartaric salts, potassium hydrogen tartrate (KHT) or calcium tartrate (CaT) is one of the major causes of instability of wines.

Tartaric acid is the main organic acid produced by the grape berry during its development. It is dissolved in the form of potassium and calcium salts into grape musts during the processing of berries. During the fermentation, the solubility of salts of tartaric acid decreases with the increase of ethanol concentration (due to the fermentation of sugars).

In young wines, potassium hydrogen tartrate (KHT) is always present in supersaturating concentrations and crystallises spontaneously. After bottling of wines, the KHT-instability may become a commercial problem due to the unpredictable character of the crystallisation. Besides, consumers often perceive the presence of crystals in the bottle as a sign of inferior quality of the wine. Physical treatments can be used prior to bottling of the wine to prevent crystallisation of tartrate salts. These treatments consist in promoting the crystallisation by cooling the wine to -4°C and subsequent removal by filtration or in elimination of the potassium and tartaric ions by electrodialysis or by the use of ion-exchange resins. However, these time- and energy-consuming processes are supposed to alter the colloidal equilibrium of wines.

The alternative to physical treatments of wines is to use additives, which prevent the nucleation and/or the growth of KHT crystals.

Carboxymethyl cellulose and meta-tartaric acid belong to the group of additives which interfere with KHT crystallisation. However, carboxymethyl cellulose is not approved as a wine additive in current regulation. Meta-tartaric acid, on the other hand, is unstable at the pH of wine and at the temperature at which wine is stored. Over time, the meta-tartaric acid will hydrolyse and its protective effect will disappear. Therefore, its use is restricted to wines for quick consumption. Another drawback is that ideally an additive should be a natural component of wine. This is definitely not the case with meta-tartaric acid or carboxymethyl cellulose.

Natural additives, which are active against both nucleation and growth rate of KHT crystals, are preferred over chemical additives.

An example of a natural additive is mannoprotein. Mannoprotein is, together with glucan, the main component of cell walls in yeasts (Lipke P.N. et al, J. Bacteriol. (1998) 180(15): 3735-3740). Mannoprotein is mainly obtained from yeast cells by two types of methods: physical methods and enzymatic methods. The most common physical method to obtain mannoprotein is that described in Peat S. et al, J. Chem. Soc. London (1961) 28-35, wherein mannoprotein is obtained by heat-extracting yeast cells at neutral pH. An enzymatic method to obtain mannoprotein from yeast is for instance described in WO 96/13571. This method comprises treatment of isolated yeast cell walls with a β-glucanase preparation and isolation of the product via ultrafiltration.

Mannoprotein may have the disadvantage that, once added to wine, it gives rise to undesirable turbidity and, in some cases, to precipitation of by-products.

EP-A-1094117 describes a method to produce soluble powders of mannoprotein wherein the problem of the turbidity caused by by-products present in mannoprotein preparations is reduced. In this method, yeast cell walls are isolated after autolysis and are subsequently subjected to a selective hydrolysis either at 95-100°C for 15-30 hours or under the action of β-glucanase. In both cases mannoprotein and other impurities are solubilised. Several steps are necessary to purify the solubilised mannoprotein.

Mannoprotein can be added to wine or wine must in the form of a powder or in the form of a solution. Use of mannoprotein in powder formulation has the advantage that such formulation is microbiologically stable in time. However in order to correctly dose the amount of a mannoprotein powder formulation in wine, mannoprotein is preferably dissolved in water or wine. To avoid diluting the wine or must too much upon addition thereto of the mannoprotein solution, said solution should have a concentration in mannoprotein of approximately 200-300 g/l. However due to the slow dissolution of the mannoprotein, preparation of such a concentrated solution starting from a powder requires continuous stirring of the solution for 1-2 hours and therefore is cumbersome for the wine maker. Furthermore during the production of the solution there is a risk of microbiological contamination thereof which is of course undesirable.

For the above-mentioned reasons, the use of a ready-to-use mannoprotein solution for the stabilisation of wine would be preferred by the wine maker. However production of a ready-to-use mannoprotein liquid formulation presents some challenges. A ready-to-use mannoprotein solution should be a translucent liquid formulation at the concentration of use, i.e. at 100-300 g/l. Furthermore the liquid formulation should be stable. In particular the activity of the mannoprotein solution as wine stabiliser against tartrate precipitation in wine should be preserved in the time period between the production of the solution by the mannoprotein manufacturer and the use thereof by the wine maker. A goal of the present invention is to provide a solution to the above-mentioned problems.

### Detailed description of the invention

The invention provides a process to produce a mannoprotein solution comprising a) subjecting a suspension of yeast cells to enzymatic hydrolysis, preferably under the action of exogenous enzymes and after inactivation of native yeast enzymes, whereby said yeast cells are degraded and mannoprotein and other yeast components are solubilised and released from the degraded yeast cells; b) recovering the solubilised mannoprotein as a solution, preferably using membrane filtration; and e) inactivating protease by subjecting the mannoprotein solution obtained after step b) heat treatment at a temperature of 70°C or higher.

The mannoprotein solution produced by the process of the invention has a turbidity, when measured by nephelometry at a concentration of 200 g/l of mannoprotein and at a pH in the range of pH 4 to pH 8, lower than 70 NTU, preferably lower than 60 NTU, more preferably lower than 50 NTU, most preferably lower than 40 NTU,

In the context of the present invention "mannoprotein" is defined according to RESOLUTION OENO 26/2004 of the OIV (Organisation Internationale de la Vigne et du Vin) as a product which can be extracted from *Saccharomyces cerevisiae* cells and/or yeast cell walls by physico-chemical or enzymatic methods. Mannoproteins are different structures depending on their molecular weight, their degree and type of glycosylation and their load size.

The applicant has surprisingly found that a very clear mannoprotein solution, clearer than any mannoprotein solution described in the prior art, can be produced even at concentrations of 200-300 g/l. This mannoprotein solution is particularly suitable to be used in wine for the stabilisation thereof against tartrate precipitation as it can be added to wine in high concentration without diluting the wine too much. Furthermore the mannoprotein solution produced by the process of the invention does not cause turbidity when added to wine. Because the turbidity of a mannoprotein solution is dependent on the concentration and on the pH, the values of turbidity are measured at a concentration of 200 g/l of the solution. However this should not be interpreted as meaning that the solution produced by the process of the invention has a fixed concentration of mannoprotein of 200 g/l, but that the turbidity thereof is measured at a concentration of 200 g/l. Therefore depending on its initial concentration the mannoprotein solution can be diluted or concentrated up to 200 g/l prior to measurement of turbidity. At a pH of 5.5 the turbidity of the mannoprotein solution produced by the process of the invention, measured by nephelometry at a concentration of 200 g/l mannoprotein, is preferably 100 NTU or lower, more preferably 50 NTU or lower, even more preferably 40 NTU or lower, most preferably 30 NTU or lower.

Proteases and β-glucanases are enzymes which can be used in the production of mannoproteins from yeast or yeast cell walls and which are (usually) present as impurities in the mannoprotein produced according to the known methods. The presence of these impurities does not represent a problem when the mannoprotein is isolated as a powder. The presence instead of even small traces of protease activity and optionally β-glucanase activity in the solution causes a fast decrease of activity of the mannoprotein solution with respect to stabilisation of wine towards tartrate precipitation.

The Applicant has surprisingly found that when a mannoprotein solution is free from protease activity, for instance alkaline protease activity, and optionally free from β-glucanase activity, the shelf life of the solution and its activity as wine stabiliser against tartrate precipitation is prolonged. The presence instead of even small traces of protease activity and optionally β-glucanase activity in the mannoprotein solution causes a fast decrease of activity of the solution in respect of stabilisation of wine towards tartrate precipitation.

The mannoprotein solution produced by the process of the invention is free of protease activity and optionally free of β-glucanase activity.

With "protease activity" it is herewith intended the total enzyme activity due to endoproteases (EC 3.4.21-24). Free of protease activity means that protease, activity in the mannoprotein solution will be lower than the minimum activity detectable with the method used to measure protease activity. Depending on the type of proteases which are expected to be present in the solution, specific methods to measure protease can be used and specific protease units can be defined. These methods are known to those skilled in the art. In Materials and Methods the method used in the present invention to determine protease activity is given and protease activity unit is defined.

With "β-glucanase" activity it is herewith intended the total enzyme activity due to β-1,3, β-1,4 and/or β-1,6 endo glucanases. Beta-glucanase activity can be determined according to the method described in Materials and Methods. Free from β-glucanase activity means that the level of β-glucanase activity in the solution is lower than the minimum activity detectable with a suitable method to measure β-glucanase activity such as that described in Materials and methods.

The mannoprotein solution produced by the process of the invention finds its preferred use in the stabilisation of wine or must. For this reason it is important that the solution is devoid of the presence of any harmful microorganism. Furthermore the presence of any viable microorganism can have an impact on the storage stability of the solution and in the preservation of its activity as wine stabiliser. For these reasons in one embodiment of the invention, the mannoprotein solution produced by the process of the invention has a total amount of viable microorganisms present in the solution, measured in Colony Forming Unit (CFU) / ml which is lower than 10, preferably lower than 5, more preferably the mannoprotein solution is sterile.

With the wording "total amount of viable microorganisms" is meant the total amount of aerobic and anaerobic psychrophilic microorganisms + aerobic and anaerobic mesophilic microorganisms + aerobic and anaerobic thermophilic microorganisms present in the solution. The total amount of viable microorganisms present in the solution, measured in Colony Forming Unit (CFU) / ml is defined in Materials and Methods and is established by determination of the total plate count (TPC), a method which is well known to those skilled in the art and is shortly described in Materials and Methods. Most preferably the mannoprotein solution is sterile, for instance the total amount of viable microorganisms present in the solution, measured in Colony Forming Unit (CFU) / ml of solution is 0. The mannoprotein solution is preferably packed in a sterile container which will be hermetically closed.

The mannoprotein solution according produced by the process of the invention is preferably an aqueous solution.

The solution produced by the process of the invention is particularly suitable to be added to wine or wine must for the stabilisation thereof. In order to avoid diluting the wine or must too much upon addition of the mannoprotein solution, the concentration of mannoprotein in the solution is preferably between 50 g/l and 500 g/l, more preferably between 100 g/l and 300 g/l, most preferably of 200 g/l.

The pH of the solution is preferably such that the stability of the mannoprotein is preserved in time and such as to avoid turbidity of the solution. Therefore the pH of the mannoprotein solution is preferably comprised between 3 and 8, more preferably between 4 and 7, most preferably between 5 and 6.

The solution produced by the process of the invention is preferably microbiologically stable in time. Therefore in a preferred embodiment at least a stabilising additive is added to the mannoprotein solution. More preferably the stabilising additive is sulphur dioxide. More preferably sulphur dioxide can be added to the solution in the form of bisulphite salt, more preferably as sodium or potassium bisulphite (K₂S₂O₅). Addition of sulphur dioxide has another advantage next to maintain the mannoprotein solution microbiologically stable. Mannoprotein solutions have a colour which can vary from light yellow to brownish. However the use of a brownish solution for the stabilisation of wine would not be very appealing to the winemaker. Therefore a mannoprotein solution to be used in wine or must should preferably be only slightly coloured. It has been found that the addition of sulphur dioxide to a mannoprotein solution can reduce the colour thereof from brownish to pale yellow. Therefore the amount of sulphur dioxide to be added to the solution is preferably in the range of 1 to 20 g/l (measured as amount of K₂S₂O₅ added):

The mannoprotein solution produced by the process of the invention, dissolved in wine, should be translucent and stable. In particular, the activity of the dissolved mannoprotein solution as wine stabiliser against tartrate precipitation in wine should be preserved in the time period between the production of the solution by the manufacturer thereof and the use of the solution by the wine maker. Therefore in a preferred embodiment the mannoprotein solution produced by the process of the invention dissolved:
- in water with a concentration of 1 g/l (based on dry weight) has a turbidity, measured at a pH of 3.5, of 4 NTU or less, preferably of 2 NTU or less, more preferably of 1 NTU or less, and / or
- in 12% v/v ethanol in water with a concentration of 1 g/l (based on dry weight) has a turbidity, measured at a pH of 3.5, of 20 NTU or less, preferably of 10 NTU or less, even more preferably of 5 NTU or less, most preferably of 1 NTU or less, and / or
- in white wine with a concentration of 0.2 g/l (based on dry weight) has a turbidity of ≤10 NTU, preferably of ≤5 NTU, even more preferably of ≤2 NTU, most preferably of ≤1 NTU.

The mannoprotein solution produced by the process of the invention is preferably characterised by a carbohydrate content in the mannoprotein of at least 50% w/w, based on the mannoprotein dry matter, of which at least 70% w/w, based on the total carbohydrate content, consists of mannose residues in the form of mannose oligomers or polymers. Preferably the mannoprotein solution produced by the process of the invention is characterised by an amount of phosphorous (measured as P₂O₅) in the mannoprotein which is lower than 1 % w/w based on dry matter, more preferably 0.8% w/w or lower, even more preferably 0.7% w/w or lower. Generally the amount of phosphorous can be as low as 0.4% w/w based on dry matter. The amount of phosphorous in the mannoprotein can be measured according to a well-known AES-ICP method (Atomic Emission Spectroscopy with the aid of Inductive Coupled Plasma).

The mannoprotein solution produced by the process of the invention may be combined with one or more other wine additives. Examples of wine additives include carboxymethyl cellulose, meta-tartaric acid and arabic gum. In a preferred embodiment, the mannoprotein solution is combined with arabic gum.

In step a) the mannoprotein is extracted from an aqueous suspension of yeast cells by subjecting the suspension of yeast cells to enzymatic hydrolysis whereby said yeast cells are degraded and mannoprotein and other yeast components are solubilised and released from the degraded yeast cells.

Any type of yeast can be used in the process of the invention. In particular, yeast strains belonging to the genera *Saccharomyces, Kluyveromyces* or *Candida* may be suitably used. Yeast strains belonging to the genus *Saccharomyces,* for example the strain *Saccharomyces cerevisiae,* are preferred.

The process of the present invention may start with a suspension of yeast cells in an aqueous liquid, for instance a fermentation broth of the yeast cells in question. Suitable fermentation processes leading to suspensions of yeast cells are known in the art. In some cases the fermentation broth can be concentrated before use in the present process, for example by centrifugation or filtration. For example, cream yeast (Baker's yeast which has been concentrated to 15-27% w/w of dry matter content) may be used:

In step a) the enzymatic hydrolysis of the suspension of yeast cells may be performed by subjecting said suspension to the action of native yeast enzymes and/or added exogenous enzymes.

The conditions used to perform the enzymatic hydrolysis are dependent on the type of enzyme used and can be easily determined by those skilled in the art. Generally, enzymatic hydrolysis will be performed at a pH between 4 and 10 and at a temperature between 40°C and 70°C degrees. Generally the enzymatic hydrolysis will be performed for a time comprising between 1 and 24 hours.

In a preferred embodiment of the invention native yeast enzymes are first inactivated and subsequently exogenous enzymes are added to the suspension. Those skilled in the art know how to inactivate native yeast enzymes. Inactivation may for example be affected by a pH treatment or a heat shock, the latter method being preferred. A heat shock can be suitably performed by treating the yeast cell suspension at a temperature of 80-97°C for 5 to 10 minutes. Once the native yeast enzymes have been inactivated, exogenous enzymes can be added to the suspension of yeast cells to perform the enzymatic hydrolysis. Preferably a protease, more preferably an endoprotease, is used for this purpose. Preferably the exogenous enzymes used to perform enzymatic hydrolysis comprise an enzyme to degrade RNA into ribonucleotides. Any RNase can be used at this regard, for instance 5'-Fdase (5'-phosphodiesterase). The enzyme used to degrade RNA into ribonucleotides can be added together with or subsequently to the treatment with the above-mentioned enzymes, for instance together with or subsequently to the treatment with protease. Optionally, a deaminase, for instance adenylic deaminase, is also used together with, or subsequently to, the treatment with the above-mentioned enzymes.

Prior to step b), the enzyme(s) used in step a) may be inactivated for instance by using methods as mentioned above.

In step b) of the process the mannoprotein is recovered as a solution, preferably using membrane filtration. The solution is optionally concentrated up to the desired final concentration.

Preferably insoluble material, for instance derived from yeast cell walls, is removed prior to recovery of the mannoprotein in step b), generally by a solid-liquid separation method, preferably by centrifugation or filtration. The mannoprotein may be recovered by any method suitable thereto.

In a preferred embodiment in step b) the mannoprotein is recovered as a solution using membrane filtration, preferably using ultrafiltration (UF) or diafiltration. Membrane filtration methods, in particular ultrafiltration or diafiltration are known to those skilled in the art. Preferably, the mannoprotein is recovered by ultrafiltration (UF) or diafiltration. In cases where UF or diafiltration is used to recover mannoprotein, filters with a molecular weight cut-off of 100 kDa or lower, preferably from 3 to 50 kDa, more preferably from 3 to 10 kDa, can be used. The mannoprotein fraction remains in the retentate resulting from the ultrafiltration or diafiltration step. When the insoluble material is not removed prior to ultrafiltration or diafiltration, the retentate comprising mannoprotein and the insoluble material can be resuspended (in solution) and the insoluble material is preferably removed, for instance using common solid-liquid separation techniques as mentioned above.

Preferably, after recovering the mannoprotein as a solution the recovered mannoprotein can be treated in step c) of the process of the invention, with an RNase, for instance Fdase (phosphodiesterase), under conditions sufficient to convert RNA residues present in the recovered mannoprotein solution into ribonucleotides. The treatment can be performed using methods known in the art. This treatment is optionally followed by one or more membrane filtration steps for instance ultrafiltration or diafiltration, (step d) of the process of the invention), in order to remove impurities of low molecular weight using membranes as those indicated above. The mannoprotein solution is recovered in the retentate.

The mannoprotein solution can undergo pH adjustment and/or concentration at any suitable step in the production process using methods known to those skilled in the art.

The mannoprotein solution produced by the process of the invention is free of protease and optionally β-glucanase activity. The latter result is achieved by step e) subjecting the mannoprotein solution obtained after step b), c) or d) to a treatment suitable to inactivate protease and optionally β-glucanase, by subjecting the solution to heat treatment at a temperature of 70°C or higher, preferably at a temperature comprised between 80°C and 140°C, wherein the heat treatment is preferably performed for a time comprised between 2 seconds and 60 minutes. Preferably step e) will be performed after step d) or after sterilisation, for instance after sterile filtration as mentioned hereafter.

The temperature used in the heat treatment is preferably between 80°C and 140°C. The duration of the heat treatment will be such that preferably all enzyme activity will be removed from the mannoprotein solution. The duration of the heat treatment will depend on the temperature used. For example lower temperatures will require a longer duration of the heat treatment while higher temperatures will require only a short time. Therefore a treatment at 80°C for 30 minutes will fall under the scope of the claim as well as a treatment at 130°C for 7 seconds. The heat treatment is preferably performed by using a UHT treatment using conditions which are commonly used in the food or beverage industry. A UHT treatment may typically be effected at a temperature of 130-145°C for a duration of time of 2-10 seconds. With a heating treatment performed at 130°C or higher for sufficient time, for example a heating treatment at 130°C for 7 seconds, a solution will be obtained which is free from protease and β-glucanase activity, and in general free of any enzymatic activity.

By using a heating step generally at least part of micro-organisms present in the mannoprotein solution will be deactivated or killed. Preferably the heating treatment is a UHT treatment as indicated above because in the latter case all microorganisms or microbial spores which might be present in the solution will be killed or deactivated.

The process of the invention may comprise subjecting the mannoprotein obtained after step b), c), d) or e) to sterilisation, preferably to sterile filtration (step f) in order to remove all microorganisms present in the solution. The latter can be achieved by using specific filters known to those skilled in the art. Preferably sterile filtration is performed after step d) or e).

Prior to or after the treatment to inactivate enzyme activity, preferably after this treatment but prior to sterile filtering, sulphur dioxide can be added to the mannoprotein solution In the desired amount. Generally addition will be performed by adding a suitable salt as indicated above. Those skilled in the art know how to perform this addition. Other stabilising additives can be added to the mannoprofein solution at any suitable step during the production process, generally at any step after step b) and prior to sterile filtration.

At the end of the production process the mannoprotein solution can be aseptically packed according to methods known to those skilled in the art. The sterile containers which can be used can be produced of several materials. Suitable containers may be sterile bags within a cardboard box. The bags may be provided with a gland which can be perforated by a screw-on tap.

It will be understood by those skilled in the art that the process of the present invention may encompass a step in which the mannoproteins solution is dried by methods known to those skilled in the art, for instance by lyophilisation or spray drying, to yield a mannoprotein in solid form, for Instance in the form of a powder or granulate. Said mannoprotein in solid form is also encompassed by the present invention.

In a second aspect the invention provides a process to stabilise wine by preventing and/or retarding the crystallisation of salts of tartaric acid wherein a mannoprotein solution in a first step is obtained from a process as defined in any of Claims 1-4 is added in a second step to wine or to grape must to be used in the production of wine. The composition is preferably added to the wine during ageing, for instance after fermentation but before bottling. The invention is extremely suitable for white wines and rose wines, but also for red wines or sparkling wines.

The mannoprotein solution is added in effective amounts to achieve a stabilising effect. Generally, it can be added to wine in a concentration between 10-2000 mg per litre of wine. Good results are already obtained by adding the solution up to a final concentration of mannoprotein in the wine of 10 to 400 mg per litre of wine. The skilled person will understand that the amount added will also depend on the addition or presence of for instance other wine stabilisers such as carboxymethyl cellulose, meta-tartaric acid or arabic gum, and on the degree of supersaturation of the KHT in the wine prior to addition.

The nucleation and crystal growth of KHT in wine can be measured and quantified by the following methods (Moutounet et al. In : Actualites Oenologiques 1999 Vieme Symposium International d'Oenologie de Bordeaux (Lonvaud-Funel ed.)).

The first method, indicative of crystal nucleation, measures the time of appearance of crystals in the wine when stored at -4°C. A visual inspection is performed daily and the time necessary to detect the appearance of crystals (Tcrys) is expressed in number of days.

The second method, indicative of crystal growth, measures the Degree of Tartaric Instability (DTI) of the wine. Hereto, wines are stirred at -4°C and the initial conductivity is measured. Subsequently, calibrated crystals of KHT are added and the conductivity is then measured after a stable value has been reached. The DTI is defined as the percentage decrease of the initial conductivity.

The third method measures the true, dissolved tartaric acid concentration. An accurate volume of the wine is transferred into a glass vial, and mixed with the same accurate volume of D₂O containing a precisely known concentration of maleic acid. The 1H NMR spectrum is run at conditions of full relaxation, and the integral of the internal standard (maleic acid) is compared with the integral of tartaric acid. In this way the dissolved tartaric acid concentration can be determined with very high precision and accuracy.

Generally, wine unstable with respect to crystallisation of tartaric acid salts has a Tcrys that can vary between 0.5 and 6 days. Stabilized wine towards crystallisation of salts of tartaric acid is obtainable by adding to wine or to grape must to be used in fermentation for the production of wine the mannoprotein solution produced by the process of the invention in a concentration suitable to prevent and/or retard the crystallization of salts of tartaric acid. Said stabilized wine is characterized by a Tcrysstabilized wine/Tcrysunstable wine of at least 2, preferably at least 5, more preferably at least 10, even more preferably between 10 and 60 as measured according to method 1.

The production of wine is characterised by the following steps which are well known to those skilled in the art: a) extracting wine must from grapes; b) subjecting the wine must to fermentation under the action of yeast to yield wine which is subsequently separated from solid residues; c) optionally subjecting the wine to aging and d) bottling or packaging the wine. After separation of wine from solid residues present after fermentation, other insolubles are generally formed in the wine in the subsequent steps of its production (for instance solids due to the precipitation of tartrate salts for instance when the wine undergoes cold stabilisation). Therefore wine production, especially if performed at large scale, very often comprises the step of filtering the wine to remove the above mentioned insoluble compounds formed just prior to bottling or packaging of the wine. Wine making can comprise, depending on the wine to be produced, additional steps to those mentioned above. All these steps are known to those skilled in the art.

In an (industrial) wine making process comprising a step for stabilising wine by addition of a wine stabiliser such as mannoprotein, said mannoprotein can be added either to the wine must or to the wine. Generally it will be added to the wine, normally during ageing and prior to filtration and bottling. In case addition of the mannoprotein occurs during aging and prior to filtration and bottling it has been surprisingly found that activity of the mannoprotein as stabiliser against precipitation of tartrate salts decreases after filtration of the wine. Therefore a higher dose of mannoprotein should be added in order to achieve the desired stabilisation in the bottled wine. A solution to this problem is therefore to add the mannoprotein in form of a solution after the last filtration step which the wine undergoes prior to bottling.

The mannoprotein solution added to the wine after filtration can be formed just prior to addition to the wine by mixing mannoprotein to water or wine or it can be a ready-to-use mannoprotein solution. Preferably the mannoprotein solution is produced using mannoprotein which is very soluble in water and wine and which does not cause any turbidity when added to wine. More preferably the mannoprotein solution used in the process to stabilise wine is a (ready-to-use) solution produced by the process of the invention. It may be combined with one or more other wine additives or stabilisers, such as carboxymethyl cellulose, meta-tartaric acid and arabic gum. In a preferred embodiment, the mannoprotein solution is combined with arabic gum.

The solution can be added after filtration to the wine in a proper amount suitable to stabilise the wine against precipitation of tartrate salts. Addition to the wine can occur either by mixing the solution with the wine prior to bottling or it can be directly added in the bottle (or package) prior to filling thereof with wine.

The present invention will be now illustrated by some examples which however do not intend to be limiting.

### EXAMPLES

### Materials and Methods

### GPC determination of the molecular weight

The molecular weight of peptides was measured by GPC with a TSKGel G2000SWxl column 7.8x300 mm (Tosoh Bioscience, Japan) using as a mobile phase a buffer 20mM NaH2PO4·H2O 100mM NaCl at pH 6.0, using a flow rate of 0.5 ml/min and detection at 214 nm. Sample injection volume was 25 µl. The column was calibrated with 4 standard globular proteins from Bio-Rad USA: Thyroglobulin 670kDa, y-globulin 158kDa, Ovalbumin 44kDa and Vitamin B12 1350 Da. A straight calibration line of log (MW) against retention time was fitted and this calibration line was used for determination of the average molecular weight of fractions. Detection was effected using UV3000 Thermo Finnigan (USA).

### Turbidity determination

Turbidity was determined with a HACH 2100 N turbidity meter (Hach-Lange, Düsseldorf, Germany) equipped with a tungsten lamp (400-600 nm) at a temperature of 20°C

### Total plate count

Three samples of mannoprotein solution were stored under the specific conditions mentioned below prior to analysis and analyzed using the total plate count (TPC) method for the possible presence of psychrophilic, mesophilic, and thermophilic microorganisms capable to grow in aerobic and anaerobic atmosphere. The determination of TPC was done by pouring about 15 ml of PCA agar (Plate Count Agar, Oxoid, UK) on 1 ml of mannoprotein solution present in a Petri dish. The TPC incubation was executed under one of the 6 conditions mentioned below. For each condition the analysis was made in duplicate. In total for each mannoprotein sample 12 Petri dishes were prepared, 6 were incubated aerobically the other 6 anaerobically.

### Conditions for determination of aerobic and anaerobic psychrophiles:

Samples storage: At 8°C for 30 days
Analysis: TPC on PCA plates
Incubation: Aerobic or anaerobic at 8°C for 7 days

### Conditions for determination of aerobic and anaerobic mesophiles:

Storage samples: At 30°C for 10 days
Analysis: TPC on PCA plates
Incubation: Aerobic or anaerobic at 30°C for 3 days

### Conditions for determination of aerobic and anaerobic thermophiles:

Storage samples: At 55°C for 10 days
Analysis: TPC on PCA plates
Incubation: Aerobic or anaerobic at 55°C for 3 days

After the proper incubation time, each Petri dish was analysed to determine the amount of colonies formed in each dish. The amount of colonies in each dish gives the Colony Forming Unit (CFU) per milliliters of solution for the specific type of microorganism. The total amount of viable microorganism measured as CFU per milliliters of mannoprotein solution as defined in this patent application is given by CFU/ml (anaerobic psychrofiles) + CFU/ml (aerobic psychrofiles) + CFU/ml (anaerobic mesophiles) + CFU/ml (aerobic mesophiles) + CFU/ml (anaerobic thermophiles) + CFU/ml (aerobic thermophiles).

### Measurement of protease activity

Free amino groups that are liberated after the action of a protease on N,N-dimethylcasein react with trinitrobenzenesulphonic acid yielding a yellow colour that can be measured spectrophotometrically at 405 nm. An enzyme standard containing alkaline protease is used for calibration. Five standard solutions are made with activities from 10 - 60 U/ml. (One U is defined as the amount of enzyme needed to liberate 7.59 µmol of p-nitroanilin per minute from suceinyl-L-alanyl-L-alanyl-L-propyl-L-phenylamine-para-nitroanaline (2.18 mg/ml) at pH 8.5 (0.1 M TRIS) and 37°C). 2 ml N,N-dimethylcasein solution (1.68 g/l dissolved in borax buffer containing 10 g/l disodiumtetraborate adjusted with 8.5% phosphoric acid to pH 8.5) is mixed with 200 µL standard- or sample solution and incubated for 15 minutes at 37°C. Subsequently 1.2 ml trinitrobenzenesulphonic acid solution (4 mM in 12 mM HCl) is added. After 5 minutes of incubation at 37°C the absorbance is measured at 405 nm. By using a calibration line, the activity of unknown samples can be calculated. The quantification limit of the test in a mannoprotein matrix is 600 U/ml.

### Measurement of beta-glucanase activity

The reduction of the viscosity of a solution of barley beta-glucan P-BGBM (Megazyme, UK) of pH 4.7 and 45°C, caused by a beta-glucanase is measured using a Grabner falling ball viscosimeter. The falling time of an iron ball in a capillary filled with substrate is a measure of the viscosity. The reduction of viscosity (decrease of the falling time) is used for calculation of the activity. An enzyme standard containing beta-glucanase activity is used for calibration. Five standard solutions are made with activities from 0.2 - 1.2 U/ml. (One unit is the amount of enzyme per ml of reaction mixture that causes a change in viscosity of the substrate with a speed giving a slope of 0.147 per minute, wherein the substrate is a solution of barley beta-glucan of pH 4.7 and 454°C): While stirring continuously, 1,1 g of beta-glucan is added to 40 ml water. 1 ml 1 M NaOH is added and the solution is stirred for 15 minutes. Subsequently the solution is incubated for 15 minutes in a boiling water bath. Then, under continuous stirring; the mixture is cooled for 30 minutes at ambient temperature. Respectively 1 ml 1M HCl and 50 ml 100% acetic acid is added. The volume is filled up with water to 100 ml. The pH of this solution should be 4.70 +/- 0.05. At 45°C, 1 ml sample (diluted in NaCl solution 10 g/l also containing 0.5 g/l BSA fraction V) is mixed with 5 ml beta-glucan substrate and incubated for 30 minutes. After 30 minutes of incubation the viscosity is measured by using the Grabner viscosimeter. By using a calibration line, the activity of unknown samples can be calculated. The quantification limit of the test in a mannoprotein matrix is 2 U/ml.

### Example 1

### Production of mannoprotein from yeast

Two litres of cream yeast from *Saccharomyces cerevisiae* was heated for 10 minutes at 95°C to inactivate the yeast own enzymes. The suspension was treated with Pescalase (commercially available serine protease from DSM Food Specialties, The Netherlands) for 6 hours at pH 6.0, 60°C. Next, the mixture was incubated with Fdase RP-1G (commercially available Fdase preparation from Amano, Japan) for 15 hours at pH 5.0, 60°C in order to hydrolyze the yeast RNA into nucleotides. The yeast extract was separated from the insoluble cell walls by means of centrifugation. The centrifugate was filtered by means of microfiltration, to remove all residual insolubles. Next, the clear filtrate was heated to 62°C and concentrated by means of ultrafiltration on a polyethersulphonmembrane (PESH, 4 kDa, NADIR, Microdyn-Nadir, Germany). FDase was added to ensure that all residual RNA was hydrolyzed. Next, the retentate was further purified by means of diafiltration at 62°C and a washing factor of 6. The retentate was concentrated to a final concentration of 200 g/l mannoprotein, and insolubles were removed by means of sterile filtration on Seitz EKS filterpads. The solution was UHT treated for 7 seconds at 130°C. The pH of the solution was 5.3. The mannoprotein solution was free from protease and β-glucanase activity. The mannoprotein solution was aseptically packed. The turbidity of the solution was measured by Nephelometry and was of 27.3 NTU. The mannoprotein solution was tested for the presence of viable microorganisms using Total Plate Count as indicated in Materials and Methods. No colonies of microorganism were detected under the tested conditions and therefore the CFU/ml of the mannoprotein solution was 0.

### Example 2

This example demonstrates what happens when UHT treatment, which is used to destroy any protease activity in the mannoprotein sample, is omitted.

### Materials and Methods:

A sterile solution of 12.5% w/v of mannoproteins was kept at 40 °C, a typical temperature for protease activity, for 11 months. A freeze-dried version of the sample was kept at room temperature for the same period of time.

The liquid product was freeze-dried first, and then stock solutions of both products were prepared at 10 mg/ml. Small volumes were added to two wines: rosé from 2006 and Chardonnay from 2005 to achieve final concentrations of 50, 100, 150, 200 and 250 mg/l. Samples were stored at -4 °C and crystal formation was monitored visually as usual. Table 1 gives the time necessary to detect the appearance of KHT crystals (days).

**Table 1**

| | Chardonnay 2005 | | Rose 2006 | |
|---|---|---|---|---|
| | Liquid kept for 11 months at 40 °C | Powder kept for 11 months at room temperature | Liquid kept for 11 months at 40 °C | Powder kept for 11 months at room temperature |
| Blank, wine | 1 | 1 | 1 | 1 |
| ± 50 mg/l | 2-3 | 4 | 4 | 7 |
| + 100 mg/l | 2-3 | 5 | 4 | 7 |
| + 150 mg/l | 4 | 8 | 6 | 7 |
| + 200 mg/l | 4 | 11 | 8 | 13 |
| + 250 mg/l | 5 | 12 | 8 | 15 |

These results show that crystals appear earlier if the mannoprotein sample is stored at 40°C, in other words, the activity of the mannoproteins is significantly reduced when the mannoproteins are stored at 40°C, a temperature which is typical for protease activity.

### Example 3

This example demonstrates the effect of enzyme activity on performance of mannoproteins.

To 20 ml of mannoprotein liquid (20% d.m.) obtained in example 1 and which is free from protease and β-glucanase activity, 100 µl of a solution of Alcalase^{®} (Sigma Aldrich, containing protease from *Bacillus licheniformis*) was added. The solution was incubated for 48 hours at pH 5.3 and 40°C. Similarly, 12.5 mg of Glucanex^{®} (Sigma Aldrich, containing β-glucanase, cellulase, protease, and chitinase activities from *Trichoderma harzianum*) was added to 20 ml of mannoproteins, and incubation was done under the same conditions. The mannoprotein solution obtained in example 1, free of enzymatic activity was incubated at 40°C, and the same solution was stored at 4°C. The mannoprotein solutions were tested in white wine supersaturated in KHT (concentration of tartaric acid 26% above saturation). Dilutions of 10 mg/ml were prepared and aliquots were added to wine in order to achieve 50, 75 and 100 mg/l of mannoproteins. The formation of crystals was monitored visually on a daily basis. Table 2 gives the time necessary to detect the appearance of KHT crystals (days).

**Table 2**

| | A | B | C | D |
|---|---|---|---|---|
| concentration mannoproteins (mg/l) | Blank 4°C | Blank 40°C | + Alcalase^{®} | + Glucanex^{®} |
| + 0 mg/l | 1 | 1 | 1 | 1 |
| +50 mg/l | 5 | 5 | 2 | 2 |
| +75 mg/l | 8 | 8 | 3 | 2 |
| +100 mg/l | 10 | 10 | 4 | 3 |

Table 2 shows that the activity of the mannoproteins is significantly reduced by the action of protease (Alcalase^{®}) and by the activity of β-glucanase, cellulase, protease, or chitinase, or by a combination of two or more of any of these activities.

The Gel Permeation Chromatogram of samples A-D was recorded as described in the Materials and Methods section. Table 3 shows the MW of the top of the peptide peak.

**Table 3: MW as determined by GPC of the top of the peptide peak**

| | A | B | C | D |
|---|---|---|---|---|
| | Blank 4°C | Blank 40°C | +Alcalase^{®} | +Glucanex^{®} |
| MW | 5.0 kDa | 5.0 kDa | 3.5 kDa | 2.3 kDa |

Table 3 shows clearly the effect of enzyme activity on the molecular weight of the mannoprotein sample.

### Example 4

### Reduction of colours means of sulphite

A stock solution of 100 mg/ml Potassium Metabisulphite K2S2O5 was prepared. To 5 ml of the mannoprotein solution obtained in example 1 increasing volumes of sulphite solution were added in order to obtain final concentrations of K2S2O5 of 0.5 g/ to 10 g/l. A first decrease of the colour was observed after 15 minutes. The colour further decreased in the course of time. After 24 days at room temperature, the colour of the solutions was measured using UV/VIS after diluting the sample 60 times. The absorbance was monitored at 450 nm. A high absorbance at 450 nm gives rise to a yelowish/brown colour. Results are shown in Table 4.

**Table 4: absorbance of mannoprotein solution at 450 nm as a function of sulphite concentration (200 g/l mannoprotein solution was diluted 60 times before UV/VIS measurement and absorbance values were multiplied by 60) and storage time.**

| | Absorbance | | | |
|---|---|---|---|---|
| **K₂S₂ O₅(g/l)** | **Day 12** | **Day 24** | **Day 120** | **Day 200** |
| **0** | 2.85 | 2.77 | 2.71 | 2.60 |
| **1** | 2.44 | 2.27 | 2.10 | 2.08 |
| **2** | 2.48 | 1.94 | 2.02 | 2.15 |
| **4** | 2.01 | 1.78 | 1.35 | 1.49 |
| **6** | 1.84 | 1.63 | 1.29 | 1.18 |
| **10** | 1.72 | 1.37 | 1.12 | 0.56 |

Table 4 shows that the absorbance of the solution and therefore the colour of the solution are significantly reduced by the addition of sulphite.

### Example 5

### Reduction of the enzymatic activity after heat treatment.

The reduction of proteolytic activity (Alcalase^{®}, Sigma Aldrich) was studied in the following experiment. 300.000 units of Alcalase^{®} were added to 1 ml of mannoprotein solution. The activity was measured before and after boiling for 10 minutes. Results are given in Table 5.

Details of the methods used to determine protease and β-glucanase activity are given in the Materials and Methods section.

**Table 5: Reduction of proteolytic activity after heat treatment**

| | Units/ml added | Units/ml recovered | Detection limit |
|---|---|---|---|
| Mannoproteins + alcalase | 300.000 | 283.000 | 600 |
| Mannoproteins + alcalase + 10 minutes 100°C | 300.000 | <600 | 600 |

It follows from Table 5 that this heat treatment reduces the proteolytic activity below the detection limit, i.e. to <0.2% of the original activity.

The same experiment was done with Glucanex^{®} (Sigma Aldrich) see table 6.

**Table 6: Reduction of glucanase activity after heat treatment**

| | Units/ml added | Units/ml recovered | Detection limit |
|---|---|---|---|
| Mannoproteins + Glucanex^{®} | 1200 | 1000 | 2 |
| Mannoproteins + Glucanex^{®} + 10 minutes 100°C | 1200 | <2 | 2 |

It follows from Table 6 that this heat treatment reduces the β-glucanase activity below the detection limit, i.e. to <0.2% of the original activity.

### Example 6

The turbidity of a liquid mannoprotein sample (LMP) prepared according to the method of the invention was determined as a function of pH and concentration using the following method:
Solutions of mannoprotein were prepared from powder as prepared in Example 1, in concentrations of 50, 100, 200, 300 and 400 g/l. In the case of 400 g/l heating was employed to achieve full solubilisation. Turbidities were measure in a HACH 2100 N turbidity meter (Hach-Lange) equipped with a tungsten lamp (400-600 nm) at a temperature of 20 °C. The pH of the samples was adjusted with HCl or NaOH solutions to pH 4.0, 5.0, 5.5, 6.0, 7.0 and 8.0.

A commercially available mannoprotein sample, Mannostab (Laffort), was dissolved in a concentration of 200 g/l, and the turbidity was also measured at pH 4.0, 5.0, 5. 5, 6.0, 7.0 and 8.0.

**Table 7: NTU values of a liquid mannoprotein sample (LMP) according to the invention and a commercial sample as a function of pH and concentration.**

| | pH 4.0 | pH 5.0 | pH 5.5 | pH 6.0 | pH 7.0 | pH 8.0 |
|---|---|---|---|---|---|---|
| | NTU | NTU | NTU | NTU | NTU | NTU |
| LMP 50 g/l | 24.9 | 23.1 | 23.9 | 24.1 | 16.0 | 13.4 |
| LMP 100 g/l | 23.6 | 19.7 | 18.6 | 17.9 | 15.1 | 14.7 |
| LMP 200 g/l | 32 | 24.3 | 22.2 | 20 | 17.0 | 15.6 |
| LMP 300 g/l | 34.6 | 26.5 | 22.8 | 20.4 | 18.9 | 15.6 |
| LMP 400 g/l | 40.2 | 26.1 | 24.7 | 20.4 | 19.5 | 15.5 |
| MannoStab 200 g/l | 1730 | 584 | 418 | 288 | 320 | n.d. |

It follows from Table 7 that the turbidity of the liquid mannoprotein preparation according to the invention is rather low at all conditions tested. The turbidity is at most 8% of the turbidity of the commercial sample. This is the case at low pH, such as pH 4, as well as at concentrated samples, such as concentrations of 400 g/l.

### Example 7

This example demonstrates the effect of adding a mannoprotein sample according to the invention after filtration of the base wine.

Sauvignon blanc and Chardonnay were clarified by means bentonite and filtered to obtain the base wine. The base wine was treated in three different ways:
1. The base wine was filtrated with cartridge filtration 0.45 µm pore size. No mannoproteins were added
2. The base wine was filtrated with cartridge filtration 0.45 µm pore size first. Then mannoproteins were added in a final concentration of 20 g/hl
3. Mannoproteins were added in a concentration of 20 g/hl to the base wine first.

Then the wine was filtrated with cartridge filtration 0.45 µm pore size. Table 8 shows the turbidity of the wines.

**Table 8 Turbidity (NTU)**

| | Blank | Mannoproteins added after filtration | Mannoproteins added before filtration |
|---|---|---|---|
| Sauvignon | 0.42 | 0.35 | 0.35 |
| Chardonnay | 0.45 | 0.47 | 0.56 |

The results show that the addition of mannoprotein delays the formation of crystals and that a mannoprotein solution produced according to the invention can be conveniently added before or after filtration of the base wine without significantly affecting its turbidity.

## Claims

1. Process to produce a mannoprotein solution which has a turbidity, when measured by nephelometry at a concentration of 200 g/l of mannoprotein and at a pH in the range of pH 4 to pH 8 lower than 70 NTU which is free of protease activity comprising a) subjecting a suspension of yeast cells to enzymatic hydrolysis, whereby said yeast cells are degraded and mannoprotein and other yeast components are solubilised and released from the degraded yeast cells; b) recovering the solubilised mannoprotein as a solution; and c) inactivating protease by subjecting the mannoprotein solution obtained after step b) to heat treatment at a temperature of 70°C or higher.

2. Process according to claim 1 further comprising the step of
d) treating the solubilised mahnoprotein recovered as a solution with phosphodiesterase.

3. Process according to claim 1 or 2 wherein in step c) the treatment is done at a temperature comprised between 80°C and 140°C.

4. Process according to any one of claims 1 - 3 wherein the mannoprotein solution obtained after step b), c), or d) is sterilised.

5. Process to stabilise wine by preventing or retarding the crystallisation of salts of tartaric acid involving
(a) the process as defined in any of claims 1- 4;
(b) adding the mannoprotein solution to wine or to grape must to be used in the production of wine.

6. Process according to claim 5 wherein the total amount of viable microorganisms present in the solution, measured in Colony Forming Unit (CFU) / ml of solution is lower than 10.

7. Process according to claim 5 or 6 wherein the solution is an aqueous solution.

8. Process according to anyone of claim 5-7 wherein the solution further comprises at least a stabilising additive.

9. Process according to anyone of claim 5-8 wherein the solution has a mannoprotein concentration between 50 g/l and 500 g/l.

10. Process according to anyone of claim 5-9 wherein the solution when dissolved in water to yield a 1 g/l (based on total dry weight) solution has a turbidity, measured at a pH of 3.5, of 4 NTU or less, and/or which when dissolved in a 12% v/v ethanol in water solution, to yield a 1 g/l solution (based on total dry weight) has a turbidity, measured at a pH of 3.5, of 20 NTU or less, and/or which when dissolved in white wine with a concentration of 0.2 g/l (based on total dry weight) has a turbidity of ≤10 NTU.

## Patentansprüche

1. Verfahren zum Herstellen einer Mannoprotein-Lösung, die eine Turbidität, wenn durch Nephelometrie bei einer Konzentration von 200 g/l Mannoprotein und bei einem pH-Wert im Bereich zwischen 4 und 8 gemessen, unter 70 NTU aufweist und frei von Protease-Aktivität ist, umfassend
a) enzymatisches Hydrolysieren einer Suspension von Hefezellen, wodurch die Hefezellen abgebaut und Mannoprotein und andere Hefebestandteile löslich gemacht und von den abgebauten Hefezellen freigesetzt werden;
b) Rückgewinnen des löslich gemachten Mannoproteins als eine Lösung; und
c) Deaktivieren von Protease durch Wärmebehandeln der nach Schritt b) erhaltenen Mannoprotein-Lösung bei einer Temperatur von 70 °C oder höher.

2. Verfahren nach Anspruch 1, ferner umfassend den Schritt
d) Behandeln des als eine Lösung rückgewonnenen löslich gemachten Mannoproteins mit Phosphodiesterase.

3. Verfahren nach Anspruch 1 oder 2, bei dem in Schritt c) die Wärmebehandlung bei einer Temperatur zwischen 80 °C und 140 °C durchgeführt wird.

4. Verfahren nach Anspruch 1 bis 3, bei dem die nach Schritt b), c) oder d) erhaltene Mannoprotein-Lösung sterilisiert wird.

5. Verfahren zum Stabilisieren von Wein durch Verhindern oder Verzögern der Kristallisation von Salzen der Weinsäure, beinhaltend
(a) das Verfahren gemäß einem der Ansprüche 1 bis 4;
(b) Hinzufügen der Mannoprotein-Lösung zu Wein oder zu bei der Produktion von Wein zu verwendendem Traubenmost.

6. Verfahren nach Anspruch 5, bei dem die in der Lösung vorhandene Gesamtmenge lebender Mikroorganismen, gemessen in koloniebildende Einheiten (KBE) / ml Lösung, weniger als 10 beträgt.

7. Verfahren nach Anspruch 5 oder 6, bei dem die Lösung eine wässrige Lösung ist.

8. Verfahren nach einem der Ansprüche 5 bis 7, bei dem die Lösung ferner zumindest ein stabilisierendes Additiv enthält.

9. Verfahren nach einem der Ansprüche 5 bis 8, bei dem die Lösung eine Mannoproteinkonzentration zwischen 50 g/l und 500 g/l aufweist.

10. Verfahren nach einem der Ansprüche 5 bis 9, bei dem die Lösung, wenn in Wasser gelöst, um eine 1 g/l Lösung (basierend auf der Gesamttrockenmasse) zu erhalten, eine Turbidität, gemessen bei einem pH-Wert von 3,5, von 4 NTU oder weniger aufweist und/oder, wenn in einer 12% v/v wässrigen Ethanol-Lösung gelöst, um eine 1 g/l Lösung (basierend auf der Gesamttrockenmasse) zu erhalten, eine Turbidität, gemessen bei einem pH-Wert von 3,5, von 20 NTU oder weniger aufweist und/oder, wenn in Weißwein mit einer Konzentration von 0,2 g/l (basierend auf der Gesamttrockenmasse) gelöst, eine Turbidität von ≤ 10 NTU aufweist.

## Revendications

1. Procédé pour produire une solution de mannoprotéines qui a une turbidité, lorsqu'elle est mesurée par néphélométrie à une concentration de 200 g/l de mannoprotéines et à un pH se trouvant dans la plage allant de pH 4 à pH 8, inférieure à 70 NTU qui est exempte d'activité protéase comprenant le fait a) de soumettre une suspension de cellules de levure à une hydrolyse enzymatique, moyennant quoi lesdites cellules de levure sont dégradées et la mannoprotéine et d'autres composants de levure sont solubilisés et libérés des cellules de levure dégradées ; b) de récupérer la mannoprotéine solubilisée sous forme de solution ; et c) d'inactiver la protéase en soumettant la solution de mannoprotéines obtenue après l'étape b) à un traitement thermique à une température supérieure ou égale à 70°C.

2. Procédé selon la revendication 1, comprenant en outre l'étape qui consiste
d) à traiter la mannoprotéine solubilisée récupérée sous forme de solution avec la phosphodiestérase.

3. Procédé selon la revendication 1 ou 2, dans lequel, dans l'étape c) le traitement est effectué à une température comprise entre 80°C et 140°C.

4. Procédé selon l'une quelconque des revendications 1 à 3, dans lequel la solution de mannoprotéines obtenue après l'étape b), c) ou d) est stérilisée.

5. Procédé pour stabiliser le vin en empêchant ou en retardant la cristallisation des sels de l'acide tartrique impliquant
(a) le Procédé tel que défini dans l'une des revendications 1 à 4 ;
(b) l'ajout de la solution de mannoprotéines au vin ou au moût de raisin à utiliser dans la production de vin.

6. Procédé selon la revendication 5, dans lequel la quantité totale de micro-organismes viables présents dans la solution, mesurée en Unité Formant Colonie (UFC)/ml de solution est inférieure à 10.

7. Procédé selon la revendication 5 ou 6, dans lequel la solution est une solution aqueuse.

8. Procédé selon l'une quelconque des revendications 5 à 7, dans lequel la solution comprend en outre au moins un additif de stabilisation.

9. Procédé selon l'une quelconque des revendications 5 à 8, dans lequel la solution a une concentration de mannoprotéines comprise entre 50 g/l et 500 g/l.

10. Procédé selon l'une quelconque des revendications 5 à 9, dans lequel la solution, lorsqu'elle est dissoute dans l'eau pour donner 1 g/l de solution (sur la base du poids sec total) a une turbidité, mesurée à un pH de 3,5, inférieure ou égale à 4 NTU, et/ou qui, lorsqu'elle est dissoute dans une solution à 12% v/v d'éthanol dans l'eau pour donner 1 g/l de solution (sur la base du poids sec total) a une turbidité, mesurée à un pH de 3,5, inférieure ou égale à 20 NTU, et/ou qui, lorsqu'elle est dissoute dans du vin blanc avec une concentration de 0,2 g/l (sur la base du poids sec total) a une turbidité ≤ 10 NTU.
